(19) Europäisches Patentamt
European Patent Office
Office européen des brevets

(11) **EP 3 384 838 A1**

(12) **EUROPEAN PATENT APPLICATION**

(43) Date of publication:
**10.10.2018 Bulletin 2018/41**

(51) Int Cl.:
***A61B 5/021*** (2006.01)     ***A61B 5/0215*** (2006.01)
***A61B 5/00*** (2006.01)

(21) Application number: **17165507.9**

(22) Date of filing: **07.04.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **MUELLER, Manfred**
  **5656 AE Eindhoven (NL)**
• **VAN DER HORST, Arjen**
  **5656 AE Eindhoven (NL)**
• **SIO, Charles Frederik**
  **5656 AE Eindhoven (NL)**

(74) Representative: **de Haan, Poul Erik et al**
  **Philips International B.V.**
  **Philips Intellectual Property & Standards**
  **High Tech Campus 5**
  **5656 AE Eindhoven (NL)**

(54) **CONSOLE, SYSTEM AND METHOD FOR MEASURING PULSE WAVE VELOCITY IN A BLOOD VESSEL**

(57)     A console for measuring pulse wave velocity of pressure waves in a blood vessel comprises a first input unit (57) arranged to receive first signals carrying information on temporal changes in blood pressure difference between two nearby locations in the blood vessel, a second input unit (59) arranged to receive second signals carrying information on pressure wave shape of pressure waves in the blood vessel, and an evaluation unit (70) configured to evaluate the first and second signals and to determine pulse wave velocity based on the temporal changes in blood pressure difference and pressure wave shape. A system for measuring pulse wave velocity of pressure waves in a blood vessel which has such a console has an interferometric sensor (12) for sensing temporal changes in blood pressure difference and a pressure wave shape sensor (62) for sensing pressure wave shape.

FIG.2

EP 3 384 838 A1

**Description**

FIELD OF THE INVENTION

The present invention relates to measuring pulse wave velocity in a blood flow in a blood vessel of a patient.

BACKGROUND OF THE INVENTION

In some medical treatments it is beneficial to measure the velocity of blood pressure or flow pulses propagating in a blood vessel of a patient. The velocity of a pressure/flow pulse is also referred to as pulse wave velocity (PWV). One example of a medical treatment in which it is beneficial to measure the pulse wave velocity is renal denervation. It is to be understood that the present invention is not limited to measuring pulse wave velocity in the renal arteries, but can be used in measuring pulse wave velocity in other blood vessels, including arteries and veins.

Renal denervation is a treatment option for resistant hypertension. In the past decade, a catheter-based RF (radio frequency) renal denervation method was developed and has gained much attention. First clinical trials showed a clear decrease of blood pressure due to the treatment. However, a larger randomized, sham-controlled, blinded trial did not show a benefit of renal artery denervation with respect to either of the efficacy endpoints for which the study was powered (reduction in office or ambulatory systolic blood pressure at 6 months). The efficacy of renal denervation is variable between patients.

Recent studies indicate that the pulse wave velocity inside the main renal artery pre-treatment is predictive of the outcome of renal denervation. The pulse wave velocity in patients with resistant hypertension can be very high, e.g. more than 20 m/s, which makes it very difficult to determine the pulse wave velocity in the relatively short renal arteries (which have a length of about 5-8 cm).

Traditionally, pulse wave velocity is determined over large distances, e.g. from brachial artery to ankle. As a pressure wave travels quickly through the vasculature (with a velocity of about 5-50 m/s), a large distance (in physiology) is needed in order to measure the required time accurately. This also means that the local pulse wave velocity in shorter arteries is traditionally not measured. In humans with resistant hypertension, the pulse wave velocity is in the higher range, which makes it very difficult to perform an accurate measurement of pulse wave velocity in the renal arteries which are usually 5-8 cm long in adults.

From the water hammer equations, the pulse wave velocity can be determined from simultaneous pressure and velocity measurements inside the artery during a reflection-free period (e.g. early systole):

$$PWV = \frac{1}{\rho}\frac{dP}{dU} \qquad (1)$$

In equation (1), P is the blood pressure, U the blood velocity, and p the blood density.

Alternatively, in case this reflection-free period cannot be used, the following relation can be used that determines the pulse wave velocity by summation over the whole cardiac cycle:

$$PWV = \frac{1}{\rho}\sqrt{\frac{\sum dP^2}{\sum dU^2}} \qquad (2)$$

However, this method relies on intravascular and simultaneous monitoring blood velocity using Doppler ultrasound and pressure sensing. Whereas intravascular pressure measurements are straightforward, the monitoring of blood velocity using an intravascular Doppler ultrasound flow device is technically challenging. Due to - amongst other factors - the flow velocity profile over the diameter of the blood vessel, the exact location of the flow sensor has a large influence on the velocity measured.

An alternative method to determine pulse wave velocity in the renal arteries is to compare the pressure signals measured by two pressure sensors which are located at different positions of the artery. If the exact distance of the pressure sensors is known, one can determine pulse wave velocity from the signal delay between the two sensors. While this solution seems straightforward in theory, it has drawbacks. Because pulse wave velocity can be very high and a distance between the sensors is limited by the length of the artery, the delay is typically very short, of the order of 0.05 ms per cm distance between the sensors. Miniaturized pressure sensors are typically not optimized for these short response times. To make a solution using two pressure sensors work, one needs to measure the pressure delay over a significant distance, typically a few cm. This makes the sensing devices relatively big, difficult to place and may hamper measuring pulse wave velocity locally. Even then noise, drift and electrical interference can make this kind of measurement difficult in practice, even though new capacitive micro-machined ultrasonic transducer (CMUT)-based sensors can resolve this kind of delay in theory.

US 2007/0201031 A1 discloses an implantable optical sensor which measures in vivo changes in blood pressure. An optical fiber waveguide in a catheter transmits light to a Mach-Zehnder interferometer. One arm of the interferometer comprises an element whose refractive index is modulated by the pressure of the blood. A light detector detects fluctuations in the light intensity arising from the transmission of external pressure fluctuations on the Mach-Zehnder interferometer. With the system and method disclosed in that document, blood pressure and maybe blood velocity can be measured but not pulse wave velocity.

Therefore, there still exists the need for devices and methods which enable accurate measurements of pulse wave velocity in a blood vessel.

SUMMARY OF THE INVENTION

It is an object of the present invention to provide a console, a system and/or a method capable of performing pulse wave velocity measurements in a blood vessel with high accuracy.

In a first aspect of the present invention, a console for measuring pulse wave velocity of pressure waves in a blood vessel is provided, comprising
a first input unit arranged to receive first signals carrying information on temporal changes in blood pressure difference between two nearby locations in the blood vessel,
a second input unit arranged to receive second signals carrying information on pressure wave shape of pressure waves in the blood vessel, and
an evaluation unit configured to evaluate the first and second signals and to determine pulse wave velocity based on the temporal changes in blood pressure difference and pressure wave shape.

The console according to the invention has input units for receiving information on temporal changes in pressure difference between two nearby locations in a blood vessel on the one hand, and further for receiving information on pressure wave shape of the pressure waves propagating in the blood vessel. Additional information about the shape of a pressure wave enables determining pulse wave velocity with high accuracy. The input first and second signals maybe optical (e.g. light intensity) and/or electrical (e.g. current/voltage) signals. The evaluation unit uses the input information on the temporal changes in pressure difference, which e.g. may be sensed by an interferometric sensor as will be described herein, and the input information on the shapes of the pressure waves, which may be sensed e.g. by a pressure sensor as will be described herein, in order to determine the pulse wave velocity from these quantities. Information on shapes of pressure waves may include specific shape features only, e.g. local extrema, of the pressure waves instead of the whole shape itself.

Since it is advantageous to sense pressure differences at two nearby locations in a blood vessel interferometrically, the first input unit may have a first photo-detector which is optically connectable to an intravascular interferometric sensor having first and second interferometer arms for insertion into the blood vessel, and the first input unit may be arranged to receive the first signals from the interferometric sensor.

Further, it is advantageous to measure pulse wave shape of pressure waves in the blood vessel optically, in particular interferometrically. To this end, the console may comprise a reference interferometer arm which is not influenced by the blood flow and optically connectable to the afore-mentioned first and second interferometer arms, wherein the reference interferometer arm may be optically connected to the second input unit.

The second input unit may comprise a second photo-detector which is optically connectable to the first and second interferometer arms and may be connected to the reference interferometer arm to detect a coherent superposition of interference light returned from the first and second interferometer arms with light returned from the reference interferometer arm.

The console may further comprise a light source for supplying light to the first and second interferometer arms and to the reference interferometer arm.

The console may further comprise one or more beamsplitters arranged between the light source and the reference interferometer arm, wherein the first beamsplitter may be configured to partially pass light from the light source to the reference interferometer arm and partially to the first and second interferometer arms.

Instead of receiving the second signals as interferometric optical signals, the second input unit may be connectable to a pressure sensor for measuring pulse wave shape at a location different from the two nearby locations.

The evaluation unit may be configured to determine from the first signals isobaric points of time of the temporal changes in pressure difference, at which the pressure difference between the two nearby locations is at least approximately zero, and to determine the pulse wave velocity using the isobaric points of time. It is advantageous to determine isobaric points of time of the temporal changes in pressure difference, because isobaric points of time of the temporal changes in pressure difference can be easily determined e.g. from interference light intensities returned from the first and second interferometer arms. At the isobaric points of time, the pressure is equal at the two nearby locations in the blood vessel.

In addition or alternatively, the evaluation unit may be configured to determine from the second signals points of time of local maxima and local minima of the shapes of the pressure waves, and to determine the pulse wave velocity using the points of time of the local maxima and local minima. For a measurement of the pulse wave velocity as accurate as possible, additional information on the pulse wave velocity is beneficial. However, it is not necessary to know exactly the whole shape of the pulse waves, but it is sufficient to know the local maxima and local minima of the shapes of the pressure waves and their time points. The computational effort for calculating the pulse wave velocity may thus be reduced significantly.

The evaluation unit may be further configured to determine the pulse wave velocity from a time delay based on the isobaric points of time and the points of time of local maxima or local minima. The pulse wave velocity may be calculated as the quotient of the known distance between the two nearby locations in the blood vessel, e.g. the distance between the first and second interferometer arms, and the time delay.

According to a second aspect, a system for measuring pulse wave velocity of pressure waves in a blood vessel is provided, comprising

an interferometric sensor configured to be inserted into a blood vessel, the interferometric sensor having an optical interferometer having a first interferometer arm and a second interferometer arm, the optical interferometer being configured to interferometrically sense temporal changes in blood pressure difference between the first and the second interferometer arms as pressure waves pass over the first and second interferometer arms,

a pressure wave shape sensor configured to sense shapes of the pressure waves, and

the above console, wherein

the first input unit is configured to receive from the interferometric sensor information on the temporal changes in blood pressure difference between the first and second interferometer arms in the blood vessel,

the second input unit is configured to receive information on pressure wave shape of pressure waves in the blood vessel from the pressure wave shape sensor.

Preferably, the optical interferometer of the interferometric sensor is a Michelson interferometer. That is both interferometer arms are terminated by a reflector, which sends the light back the way it came from through the arms. Using a Michelson interferometer has multiple advantages compared to other interferometer designs such as Mach-Zehnder interferometers. Since the light passes the pressure-sensing region of the interferometer arms twice, a Michelson-type interferometer is twice as sensitive to pressure as a Mach-Zehnder interferometer of the same material and dimension. In addition, a Michelson interferometer uses the same optical element to join the light beams that is used to separate the light beams, while a Mach-Zehnder interferometer uses different optical elements. This makes a Michelson interferometer much less expensive. This is especially important, when the interferometer is part of a disposable intravascular device, as preferred in the present invention. It also makes the interferometer smaller, which is important for intravascular sensors that have to fit to human blood vessels.

Both interferometer arms of the interferometric sensor may have light transparent structures configured and arranged to be sensitive to pressure changes. The measured temporal changes in pressure difference may be used to determine pulse wave velocity with high accuracy.

The light transparent structures may be configured as optical waveguides or optical fibers, for example. The light supplied into the first and second interferometer arms may be coherent light.

An optical property of the first and second light transparent structures may change under pressure changes acting on the first and second interferometer arms. The optical property may be a refractive index of the material of the light transparent structures. The material of the light transparent structures can be chosen in dependence on the desired sensitivity of the first and second interferometer arms to pressure changes. The first and second interferometer arms may be fully or partially in communication with the blood flow such that the blood pressure may produce a change of the refractive index in or around the first and second light transparent structures via the elasto-optic effect.

Interference light returned from the interferometer arms may be recombined into for example an optical fiber which may be the same fiber through which light is supplied to the first and second interferometer arms.

The interferometric sensor may be integrated into or integral with an interventional device, like a catheter, guide wire or the like. The interferometric sensor may be arranged at a distal end of the interventional device. The interventional device may also comprise an optical fiber as mentioned above for supplying light from a light source to the first and second interferometer arms and for returning interference light therefrom to a photodetector.

The first and second interferometer arms may be arranged to extend transversely to the blood flow when inserted into the blood vessel. Further, the first and second interferometer arms may be arranged parallel with respect to one another. When the interferometer arms are arranged perpendicular to the propagation direction of the pressure waves, the wave fronts of the pressure waves will pass first the one arm and then the other arm. The two nearby locations or the first and second interferometer arms may be spaced apart from one another in direction of the blood flow or transverse to the interferometer arms by a distance of a few cm or less, e.g. about 3 cm, about 2 cm, about 1 cm, or about 0.5 to 1 cm.

Alternatively to the configuration of the optical interferometer as a Michelson interferometer, the optical interferometer may be configured as a Mach-Zehnder interferometer. The first and second interferometer arms may be arranged in optical communication at one end with a common first light guide and at the other end with a common second light guide.

The pressure wave shape sensor may have a reference interferometer arm which is not influenced by the blood flow and arranged in optical communication with the first and second interferometer arms. This embodiment is advantageous in terms of costs and size of the interventional device, e.g. a catheter.

The reference interferometer arm may have a light transparent structure, e.g. an optical wave guide or an optical fiber, or may be configured as free space optics. The reference interferometer arm may be arranged in the console which may also include a light source for simultaneously supplying light to the first and second interferometer arms and to the reference interferometer arm.

The console of the system may further comprise a first photo-detector in optical communication with the first and second interferometer arms to detect interference light returned only from the first and second interferometer arms.

Interference light as used herein is understood as a coherent superposition of light. For example, interference light returned from the first and second interferometer arms is a coherent superposition of light returned from the first interferometer arm with light returned from the second interferometer arm.

The console of the system may further comprise a second photo-detector in optical communication with the first and second interferometer arms and with the reference interferometer arm to detect a coherent superposition of interference light returned from the first and second interferometer arms with light returned from the reference interferometer arm.

In another embodiment, the pressure wave shape sensor may have a pressure sensor separate from the interferometric sensor, which may be a non-optical sensor.

Such a separate pressure sensor may be configured as a capacitive or piezoresistive pressure sensor. The separate pressure sensor may also simply be a fluid-filled hollow catheter in communication with an external pressure sensor. The separate pressure sensor may be placed in the blood vessel system of a patient at a location remote from the location where the pulse wave velocity is actually measured. For example, the separate pressure sensor may be placed in the aorta, while the interferometric sensor may be placed in a renal artery for locally measuring pulse wave velocity there.

The evaluation unit for determining pulse wave velocity from the sensed temporal changes in pressure difference and from the sensed shapes of the pressure waves may be configured as described herein.

The isobaric points may be determined as points in time where the temporal intensity profile of the interference light returning from the first and second interferometer arms has a defined level, which may be the maximum intensity or an intermediate intensity, e.g. the half maximum intensity.

Further, the evaluation unit may be configured to determine the points of time of local maxima and local minima of the shapes of the pressure waves from a first temporal intensity profile of a first interference light returning from the first and second interferometer arms, and from a second temporal intensity profile of a second interference light returning and combined from the first, second and the reference interferometer arms, by calculating a temporal intensity profile interference light would have when returned and combined from the first interferometer arm and the reference interferometer arm alone.

In this way, the local maxima and local minima and their points of time are not directly measured, but derived from the interferometric measurements performed with the first, second and reference interferometer arms.

In a third aspect of the present invention, a method of determining pulse wave velocity in a blood flow in a blood vessel is provided, comprising

optically sensing temporal changes in pressure difference between two locations in the blood vessel which are spaced apart from one another in direction of the blood flow,

sensing pressure wave shapes of the pressure waves, and

calculating pulse wave velocity from the temporal changes in pressure difference and the shapes of the pressure waves.

The claimed method has similar and/or identical preferred embodiments as the claimed device and the claimed system as outlined above and as defined in the dependent claims.

In a fourth aspect of the present invention, a computer program is provided comprising program code means for causing a computer to carry out the steps of the method according to the third aspect when said computer program is carried out on a computer.

BRIEF DESCRIPTION OF THE DRAWINGS

These and other aspects of the invention will be apparent from and elucidated with reference to the embodiments described hereinafter. In the drawings:

Fig. 1 shows a system for determining pulse wave velocity of pressure waves in a blood flow in a blood vessel in a schematic representation;
Fig. 2 shows components of the system in Fig. 1 in a schematic representation;
Fig. 3 shows a schematic cross section of an interferometric sensor of the system in Fig. 1 to illustrate the operation principle of measuring pressure differentials;
Fig. 4 shows a typical blood pressure curve where pressure is measured at two locations with a time difference in-between;
Fig. 5 shows an example interferometric measurement of interference light intensities for the pressure curve in Fig. 4;
Fig. 6 shows another embodiment of an interferometric sensor; and
Fig. 7 shows a further embodiment of an interferometric sensor.

DETAILED DESCRIPTION OF THE INVENTION

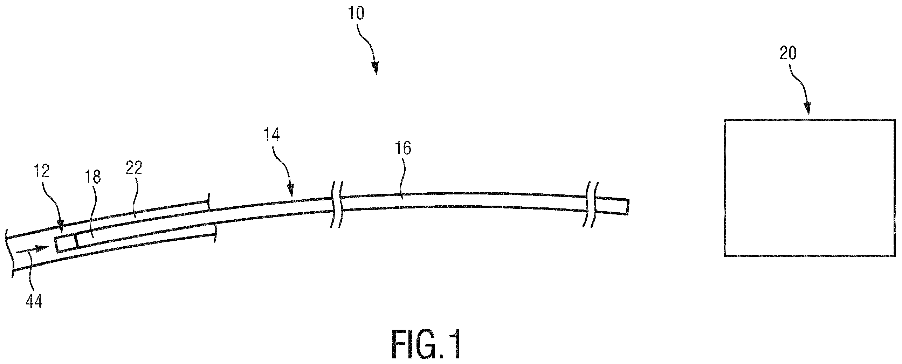

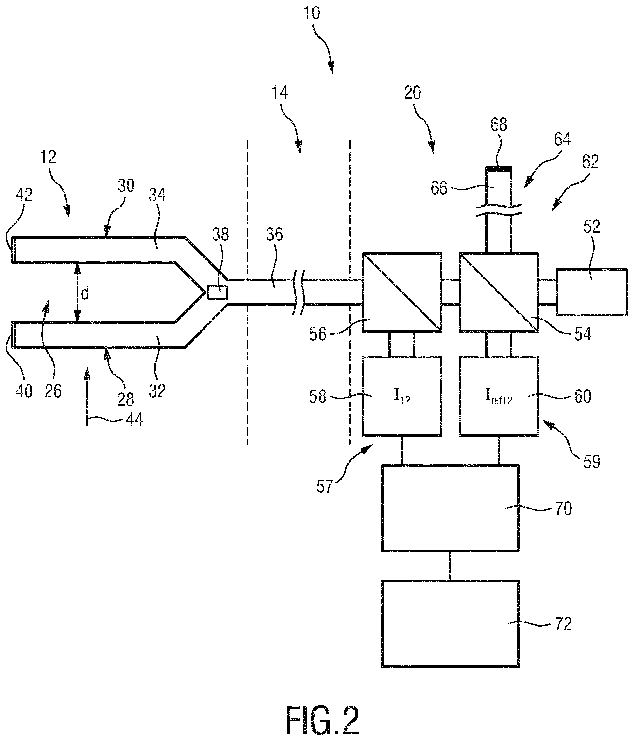

With reference to Figs. 1 and 2, a system 10 for determining pulse wave velocity of pressure waves in a blood flow and a blood vessel will be described.

The system 10 comprises an interferometric sensor 12. The system 10 further comprises an interventional device 14. The interventional device 14 may be a catheter, a guidewire or the like suitable for being introduced into a blood vessel of a patient. The interventional device 14 comprises an elongated shaft 16 which may have a length of more than 1 m. The interferometric sensor 12 may be arranged at a distal end 18 of the interventional device 14. The interferometric sensor 12 maybe integrated into or integral with the interventional device 14.

The system 10 further comprises a workstation or console 20, to which the interventional device 14 maybe connected for communication, in particular optical communication of one or more console components to be described herein with the interferometric sensor 12. The interferometric sensor 12 and a part of length of the interventional device 14 are configured to be insertable into a blood vessel 22.

With reference to Fig. 2, the interferometric sensor 12 is described next. The interferometric sensor 12 is configured to be inserted into a blood vessel like the blood vessel 22 in Fig. 1. The interferometric sensor 12 has an optical interferometer 26. In the embodiment shown in Fig. 2, the optical interferometer 26 is a Michelson interferometer.

The interferometer 26 has a first interferometer arm 28 and a second interferometer arm 30.

The first interferometer arm 28 has a first light transparent structure 32. The second interferometer arm has a second light transparent structure 34. The light transparent structures 32 and 34 may be integrated optical waveguides, optical fibers or the like. The first and second interferometer arms 30 and 32 are arranged for optical communication with a light guide 36. The light guide 36 which extends through the interventional device 14 maybe an optical waveguide or an optical fiber. Light supplied through the light guide 36 is split, e.g. by means of an integrated optical beam splitter 38 into the first and second light transparent structures 32 and 34. The light supplied through the light supply 36 maybe coherent light, e.g. emitted by a laser.

A reflector 40 is arranged at an end of the first light transparent structure 32 which end is opposite to an end of the light transparent structure 32 which is arranged in optical communication with the light supply 36. A reflector 42 is arranged at an end of the light transparent structure 34 which end is opposite to an end of the light transparent structure 34 which is arranged in optical communication with the light supply 36. The reflectors 40 and 42 of the light transparent structures 32, 34 may be e.g. integrated optical fiber-Bragg mirrors or reflective coatings or any other reflective structures.

When the interferometer 26 is inserted into a blood vessel, the interferometer arms 28, 30 extend transversely, preferably perpendicular, to the blood flow. In Figs. 1 and 2, the blood flow direction is indicated by an arrow 44. Further, the interferometer arms 28, 30 are arranged parallel with respect to one another. The interferometer arms 28, 30 are spaced apart from one another in direction transversely to their extension. In Fig. 2, the distance between the interferometer arms 28, 30 is denoted by d.

When the interferometer 26 is inserted into a blood vessel of a patient, the first and second interferometer arms 28, 30 are exposed to the blood flow. Further, one or both of the light transparent structures 32, 34 are sensitive to pressure changes. A pressure wave propagating in direction of the arrow 44 will first pass over the interferometer arm 28 and then over the interferometer arm 30 or vice versa, depending on the direction of blood flow. When a pressure wave passes over the interferometer arm 28 and then over the interferometer arm 30, the interferometer arms 28, 30 may experience different pressures over time. Preferably both the first and second light transparent structures 32, 34 are sensitive to such pressure changes, wherein an optical property of the first and second light transparent structures 32, 34 changes under the pressure changes caused by the propagating pressure wave successively passing over the first and second light transparent structures 32, 34.

The optical property which changes under pressure changes may be a refractive index of the material of the light transparent structures 32, 34. The refractive index of the light transparent structures 32, 34 may change according to the elasto-optic effect.

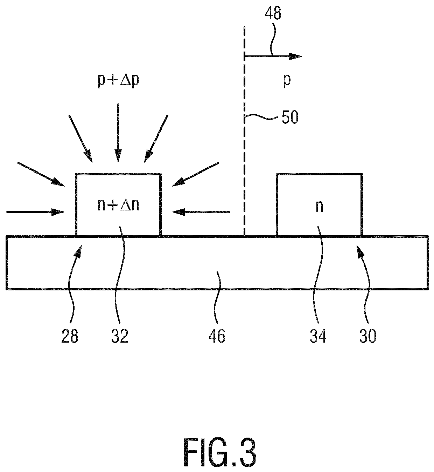

With reference to Fig. 3, the principle behind measuring pressure differences with an integrated optical interferometer like the interferometer 26 is described. Fig. 3 shows a cross-section of the two interferometer arms 28, 30, and in addition a substrate 46 on which they are fixed. When the interferometer arms 28, 30 are exposed to a blood flow (arrow 48), the pressure of the blood is communicated as pressure on the light transparent structures 32, 34.

In general, pressure exerted on an optical material will cause a change of the refractive index n of the material by via the elasto-optic effect:

$$\Delta n = n^3 \gamma S = n^3 \gamma P/E, \qquad (3)$$

wherein n is the refractive index, $\Delta$ is the elasto-optic coefficient, $S = P/E$ is the strain induced in a material with the Young's modulus E by the pressure P.

In Fig. 3, a broken line 50 indicates a wave front of a propagating pressure wave. When the pressure wave propagates in direction of the arrow 48 and passes over the first interferometer arm 28 before reaching the second interferometer arm 30, the light transparent structure 32 experiences a higher pressure $P + \Delta P$ than the light transparent structure 34, leading to a change of refractive index of the light transparent structure from n to $n + \Delta n$, while the light transparent structure 34 still experiences the pressure P and the refractive index is n. When the pressure wave fully or partially reaches the second interferometer arm 30 while having partially or fully left the first interferometer arm 28, the refractive index of the first light transparent structure 32 may be $n + \Delta n_1$, and the refractive index of the second light transparent structure 34 may be $n + \Delta n_2$, wherein $\Delta n_1$ and $\Delta n_2$ may be zero, may be non-zero and equal, or may be different from one another depending on the actual pressure exerted on the first and the second interferometer arms 28, 30.

In a symmetric interferometer, i.e. when both interferometer arms have the same optical path length, there will be no phase difference between light from the two interferometer arms, if no outside pressure is exerted. Therefore, the light from both interferometer arms will interfere constructively. If a pressure wave passes the interferometer as shown in Fig. 3, there will be a rise in blood pressure in the interferometer arm 28 ahead of a rise in blood pressure in interferometer arm 30. Therefore, the refractive index in interferometer arm 28 will transiently be different from the refractive index in interferometer arm 30, leading to a phase difference between light propagating in the light transparent structures 32 and light propagating in the light transparent structure 34, which will, when the light from both arms 28, 30 is combined (superimposed), change the intensity of the interference light. Consequently, one can use an interferometer to measure temporal changes in pressure difference between the two interferometer arms 28, 30.

With reference to Fig. 2 again, the system 10 will be further described.

The console 20 comprises a light source 52 which may be a laser. The light source 52 preferably emits coherent light.

The console 20 further comprises a first input unit 57 and a second input unit 59. The first input unit 57 is configured to receive first signals carrying information on temporal changes in blood pressure difference between two nearby locations in the blood vessel. The two nearby locations are the locations where the two interferometer arms 28, 30 are placed in the measurement. The second input unit 59 is configured to receive second signals carrying information on pressure wave shape of pressure waves in the blood vessel as described hereinafter.

The first input unit 57 comprises a first photo-detector 58 and the second input unit 59 comprises a second photo-detector 60. The console 20 further comprises a first beam splitter 54 and a second beam splitter 56. The second beam splitter 56 splits the light returning from the interferometer 26 through the light guide 36 and directs a part of the returned light to the first photodetector 58.

The system 10 further comprises a pressure-wave shape sensor 62 configured to sense shapes of the pressure waves in the blood vessel. In the present embodiment, the pressure-wave shape sensor 62 has a reference interferometer arm 64 arranged in the console 20. The reference interferometer arm 64 of the present embodiment has a light transparent structure 66. The light transparent structure 66 may be an integrated optical waveguide, an optical fiber or the like. The reference arm 64 may in other embodiments be configured as free space optics. At a free end of the reference arm 64, a reflector 68 is arranged. The reference arm 64 is not influenced by the blood flow.

The reference arm 64 may also comprise a phase shifting element to compensate for drift in the interferometer arm and to enhance the phase contrast between the interferometer arm 64 and the interferometer arms 24 and 28.

Light emitted by the light source 52 is partially transmitted into the reference interferometer arm 64 via the beam splitter 54. Thus, light emitted by the light source 52 is partially passed into the reference arm 64, and partially passed to the second beam splitter 56 which passes the light to the light guide 36 for supplying the light to the interferometer arms 28,30.

Light returned from the interferometer arms 28, 30 will be combined back into the light supply 36 via the integrated optical beam splitter 38 and will be transmitted back to the console 20. Inside the console 20, the beam splitter 56 will split off part of the light returned from the interferometer arms 28, 30 towards the first photo-detector 58 (first input unit 57) that detects an intensity $I_{12}$ of the interference light between the interferometer arms 28, 30. The rest of the light will be combined with the light returning from the reference arm 64 via the beam splitter 54, thus producing interference light from the interferometer arms 28, 30 and the reference arm 64. The beam splitter 54 passes this interference light to the second photodetector 60 (second input unit 59), and an intensity $I_{ref12}$ of this interference light is read out with the photo-detector 60. In order to obtain an interference pattern of a quality as best as possible, the optical path length of the reference arm 64 should be comparable to the optical path length of the light guide 36 through the interferometric sensor 12.

The console further comprises an evaluation unit 70 arranged in the console 20. The evaluation unit 70 may be a processor (hardware) or configured as software.

The evaluation unit 70 evaluates the signals received by the first and second input units 57, 59. In an embodiment, the evaluation unit 70 evaluates the detector signals of the photodetectors 58 and 60, as will be described herein.

The evaluation unit 70 is configured to determine the pulse wave velocity from the temporal changes in pressure difference as sensed by the interferometric sensor 12 and the shapes of the pressure waves as sensed by the pressure wave shape sensor 62, as will be described herein. A display 72 is arranged to display the pulse wave velocity as measured.

Instead of the reference arm 64, the pressure wave shape sensor 62 may have a separate pressure sensor (not shown) connectable to the console 20, especially the second input unit 59. Such a separate pressure sensor may be configured as a capacitive or piezoresistive pressure sensor. The separate pressure sensor may also simply be a fluid-filled hollow catheter in communication with an external pressure sensor. The second input unit may receive electrical signals carrying information on the pressure wave shape from the separate pressure sensor. The separate pressure sensor maybe placed at a different location in the blood vessel system of a patient than the interferometric sensor 12. For example, the separate pressure sensor maybe placed in the aorta. It does not need to be in the same location where the interferometric sensor 12 locally measures the changes in pressure difference in the blood flow as long as the changes in the pressure waveform shape between the pressure sensor and the pressure difference sensor are small.

In the following, with reference to the system 10, embodiments of a method of determining pulse wave velocity in a blood flow will be described.

The interferometric sensor 12 senses, as described above, the temporal changes in pressure difference between the first and second interferometer arms 28, 30.

However, knowing the temporal changes in pressure difference is not sufficient to determine pulse wave velocity. For determining pulse wave velocity, information about the shape of the pressure wave is needed. In the embodiment shown in Fig. 2, the shape of the pressure wave may be sensed using the reference arm 64 that is not influenced by the pressure wave. Alternatively, the shape of the pressure wave may be sensed by a separate pressure sensor as mentioned above.

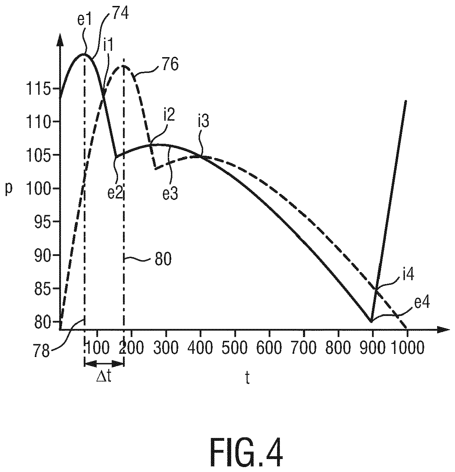

It is not necessary to sense the full shape of the pressure wave with absolute precision. It is sufficient to time the local extrema of the pressure wave, i.e. the local minima and local maxima of the pressure wave. Each local minimum and local maximum in the pressure wave corresponds with an isobaric point in the pressure difference curve as will be explained with reference to Fig. 4.

Fig. 4 shows an example of a typical blood pressure curve measured at two different locations in a blood vessel with a time delay $\Delta t$ in-between. Fig. 4 shows a blood pressure curve $P_1(t)$ denoted with reference numeral 74 (solid line) measured at a first location in the blood vessel (the pressure P and the time t are in arbitrary units). A second curve $P_2(t)$ denoted with reference numeral 76 (broken line) has been measured with a time delay $\Delta t$ at a second location different from the first location where the first pressure curve 74 has been measured. This can also be understood as the propagation of the pressure wave from the first location, e.g. the first interferometer arm 28 (as indicated with a vertical line 78) to the second location, e.g. the location of the second interferometer arm 30 (as indicated by a vertical line 80), wherein $\Delta t$ is the time the pressure wave needed to propagate from the first location to the second location.

In the blood pressure curve there are points $i_2$, $i_3$, $i_4$ at which the first pressure curve 74 intersects the second pressure curve 76. These points are isobaric points at which the pressure exerted on the first interferometer arm 28 equals the pressure exerted on the second interferometer arm 30. Further, there are points of local extrema in the pressure curve 74 which are denoted with $e_1$ (local maximum), $e_2$ (local minimum), $e_3$ (local maximum), $e_4$ (local minimum). Each local minimum and local maximum in the pressure wave corresponds with an isobaric point in the pressure difference curve (see Fig. 4).

If one takes the points of time of local maxima and local minima, i.e. $t_{e1}$, $t_{e2}$, $t_{e3}$, ..., $t_{eN}$, and the points of time of the isobaric points, i.e. $t_{i1}$, $t_{i2}$, $t_{i3}$, ..., $t_{iN}$, it is possible to determine the time delay $\Delta t$ with which the pressure wave passes from the first interferometer arm 28 to the second interferometer arm 30:

$$\Delta t = \frac{1}{N} \sum_{n=1}^{N} (t_{en} - t_{in}) \qquad (4)$$

In equation (4), $t_{en}$ is a point of time of a local extremum of the pressure wave which is an immediate neighbor of the isobaric point of time $t_{in}$. The time delay $\Delta t$ thus is calculated as the averaged or mean difference between immediately neighboring isobaric points of time $t_{in}$ and points of time $t_{en}$ of local maxima or local minima.

From the time delay $\Delta t$ according to equation (4), and the known distance d (Fig. 2) between the first and second interferometer arms 28, 30, the local pulse wave velocity can be easily calculated as

$$\mathrm{PWV} = d/\Delta t \qquad (5)$$

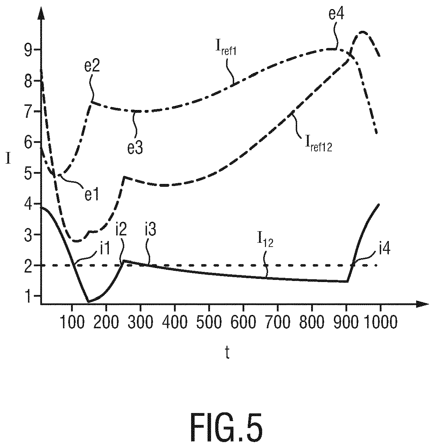

For determining the isobaric points of time of the temporal changes in pressure difference from the interferometric measurement, the evaluation unit 70 reads out the photodetector 58 which detects interference light returned from the first and second interferometer arms 28, 30 only. For determining the points of time of local maximal and local minima of the pressure waves from the interferometric measurement, the evaluation unit 70 reads out the photodetector 60 which detects interference light, which is a superposition of the interference light returned from the first and second interferometer arms 28, 30 with light returned from the reference interferometer arm 64, as will be further explained with reference to Fig. 5. Fig. 5 is an example measurement based on the pressure curve in Fig. 4 with an example evaluation.

Fig. 5 shows a measured intensity curve $I_{12}$ over time (intensity and time in arbitrary units) as detected by photodetector 58, and a measured intensity curve $I_{ref12}$ as detected by photodetector 60.

The evaluation unit 70 may determine the isobaric points of time $t_{i1}, t_{i2}, ..., t_{iN}$ from the intensity curve $I_{12}$. The isobaric points of time $t_{i1}, t_{i2}, ..., t_{iN}$ can be determined easily as they will correspond to a certain intensity (maximal intensity for a base phase difference of 0°, half intensity for a base phase difference of 90°, the latter is shown in Fig. 5). If the interferometer 26 is so sensitive that the phase change can exceed 180°, the fringes have to be counted instead.

The evaluation unit 70 may determine the points of time of local minima and local maxima of the pressure wave by using the intensity curve $I_{12}$ and the intensity curve $I_{ref12}$ together, in order to calculate what the intensity $I_{ref1}$ of the interference between the first interferometer arm 28 and the reference arm 64 only would be. The intensity curve $I_{ref1}$ is also shown in Fig. 5. The intensity curve $I_{ref1}$ thus, is a calculated intensity curve. From the intensity curve $I_{ref1}$ the shape of the pressure wave may be easily reconstructed. It is not necessary to reconstruct the pressure curve itself as a whole. It is sufficient to detect the local extrema of the pressure wave. The local extrema of the pressure wave correspond to the local extrema of the intensity curve $I_{ref1}$, if the phase changes are smaller than 180°.

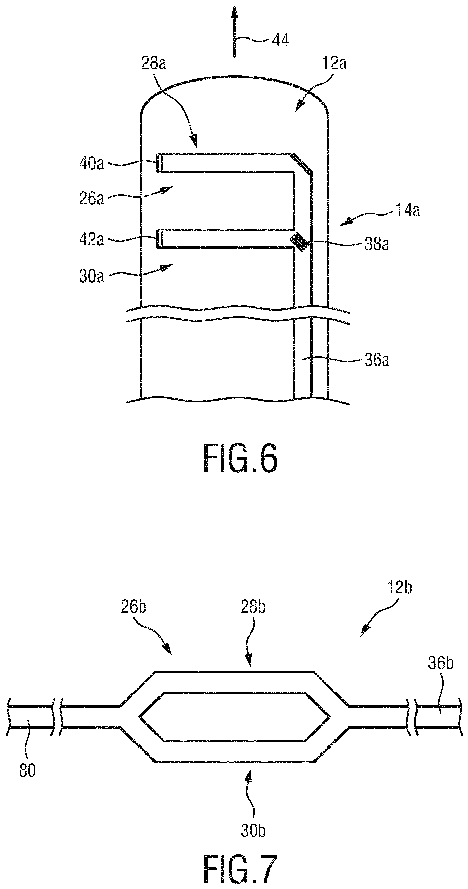

Fig. 6 shows another embodiment of an interferometric sensor 12a, wherein elements of the device 12a which are identical, similar or comparable with elements of the device 12 in Fig. 2 are denoted with the same reference numerals supplemented with 'a'.

The interferometric sensor 12a is integrated into or integral with an interventional device 14, e.g. a guide wire. The device 12a comprises an optical interferometer 26a having first and second interferometer arms 28a, 30a which may be configured like the interferometer arms 28, 30 of the device 12 in Fig. 2.

Instead of an integrated optical beam splitter 38, the device 12a has a semi-refractive grating 38a configured to split light coming from the light supply 36a into the first and second interferometer arms 28a, 30a.

Another embodiment of an interferometric sensor 12b is shown in Fig. 7. The interferometric sensor 12b has an optical interferometer 26b which is configured as a Mach-Zehnder-type interferometer. Instead of using reflectors like the reflectors 40 and 42 in the interferometer arms 28, 30 to reflect the light back in the light supply 36, the device 12 according to Fig. 7 recombines both interferometer arms 28b, 30b into a further light transparent structure 80 for returning back the interference light from the interferometer arms 28b, 30b to the console 20.

The position of the beam splitters 56, 54 and the photodetectors 58, 60 (Fig. 2) are adjusted accordingly.

A person skilled in the art will realize that there may be several modifications to the embodiments described above within the scope of the attached claims. For example, an optical isolator in front of the light source 52 could be used. A beam dump could be used after the second beam splitter 56 to trap light that is split off and not used. Further, it is possible to replace, for example, the optical beam splitters 54 and 56 with optical circulators.

An additional phase delay could be implemented in the interferometer arms 28, 30 and/or 64. For example, the reflector 68 in the reference arm 64 could be made movable to guarantee a certain phase difference between the interferometer arms 28, 30 and the reference arm 64.

The integrated optical beam splitter 38 in Fig. 2 could be replaced with a grating (as shown in Fig. 6) or with other types of beam splitters. Further, as mentioned above, the reference arm 64 could be replaced by another modality which measures the shape of the pulse waves independently. This can be done, for example, via a hollow catheter in the aorta, which will often be employed anyhow during invasive pressure measurements. This could also simplify the analysis part. If there is too much dampening between the position of the pressure measurement and the position of the interferometer like the interferometer 26, problems may arise so that it can be beneficial to add a detection algorithm for dampening to the console 20 that alerts the user if the pulse wave velocity measurement might be unreliable.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Console for measuring pulse wave velocity of pressure waves in a blood vessel, comprising:

    a first input unit (57) arranged to receive first signals carrying information on temporal changes in blood pressure difference between two nearby locations in the blood vessel,
    a second input unit (59) arranged to receive second signals carrying information on pressure wave shape of pressure waves in the blood vessel, and
    an evaluation unit (70) configured to evaluate the first and second signals and to determine pulse wave velocity based on the temporal changes in blood pressure difference and pressure wave shape.

2. Console of claim 1, wherein the first input unit (57) has a first photo-detector (58) which is optically connectable to an interferometric sensor (12) having first and second interferometer arms (28, 30) for insertion into the blood vessel, the first input unit is arranged to receive the first signals from the interferometric sensor (12).

3. Console of claim 2, comprising a reference interferometer arm (64) which is not influenced by the blood flow and optically connectable to the first and second interferometer arms (28, 30), the reference interferometer arm (64) being optically connected to the second input unit.

4. Console of claim 3, wherein the second input unit (59) comprises a second photo-detector (60) which optically connectable to the first and second interferometer arms (28, 30) and connected to the reference interferometer arm (64) to detect a coherent superposition of interference light returned from the first and second interferometer arms (28, 30) with light returned from the reference interferometer arm (64).

5. Console of claim 3, further comprising a light source (52) for supplying light to the first and second interferometer arms (28, 30) and to the reference interferometer arm (64).

6. Console of claim 2, wherein the second input unit (59) is connectable to a pressure sensor for measuring pulse wave shape at a location different from the two nearby locations.

7. Console of claim 1, wherein the evaluation unit (70) is configured to determine from the first signals isobaric points of time ($t_{i1}$, $t_{i2}$, $t_{i3}$, ..., $t_{iN}$) of the temporal changes in pressure difference, at which the pressure difference between the two nearby locations is at least approximately zero, and to determine the pulse wave velocity using the isobaric points of time ($t_{i1}$, $t_{i2}$, $t_{i3}$, ..., $t_{iN}$).

8. Console of claim 7, wherein the evaluation unit (70) is configured to determine from the second signals points of time ($t_{e1}$, $t_{e2}$, $t_{e3}$, ..., $t_{eN}$) of local maxima and local minima of the shapes of the pressure waves, and to determine the pulse wave velocity using the points of time ($t_{e1}$, $t_{e2}$, $t_{e3}$, ..., $t_{eN}$) of the local maxima and local minima.

9. Console of claim 8, wherein the evaluation unit (70) is configured to determine the pulse wave velocity from a time delay (At) based on the isobaric points of time ($t_{i1}$, $t_{i2}$, $t_{i3}$,..., $t_{iN}$) and the points of time ($t_{e1}$, $t_{e2}$, $t_{e3}$, ..., $t_{eN}$) of local maxima or local minima.

10. System for measuring pulse wave velocity of pressure waves in a blood vessel, comprising:

an interferometric sensor (12) configured to be inserted into a blood vessel, the interferometric sensor (12) having an optical interferometer (26) having a first interferometer arm (28) and a second interferometer arm (30), the optical interferometer (26) being configured to interferometrically sense temporal changes in blood pressure difference between the first and the second interferometer arms (28, 30) as pressure waves pass over the first and second interferometer arms (28, 30),
a pressure wave shape sensor (62) configured to sense shapes of the pressure waves, and
the console of claim 1, wherein

the first input unit (57) is configured to receive from the interferometric sensor (12) information on the temporal changes in blood pressure difference between the first and second interferometer arms (28, 30) in the blood vessel, and
the second input unit (59) is configured to receive from the pressure wave shape sensor (62) information on pressure wave shape of pressure waves in the blood vessel.

11. System of claim 10, comprising a reference interferometer arm (64) which is not influenced by the blood flow and optically connected to the first and second interferometer arms (28, 30), the reference interferometer arm (64) being arranged in the console (20).

12. System of claim 10, wherein the pressure wave shape sensor (62) has a pressure sensor separate from the interferometric sensor (12), the pressure sensor being configured to sense blood pressure at location remote from the interferometric sensor (12).

13. System of claim 10, wherein the first and second interferometer arms (28, 30) each comprise light transparent structures sensitive to the pressure in the blood vessel, the first and second interferometer arms (28, 30) being arranged such that a refractive index of both light transparent structures is modulated by the pressure in the blood vessel.

14. Method of determining pulse wave velocity in a blood flow in a blood vessel, comprising:

optically sensing temporal changes in pressure difference between two locations in the blood vessel which are spaced apart from one another in direction of the blood flow,
sensing pressure wave shapes of the pressure waves, and
calculating pulse wave velocity from the temporal changes in pressure difference and the shapes of the pressure waves.

15. Computer program comprising program code means for causing a computer to carry out the steps of the method as claimed in claim 14 when said computer program is carried out on the console of claim 1.

EP 3 384 838 A1

FIG.1

FIG.2

p+△p

48

p

50

n+△n

n

28    32         46        34    30

FIG.3

FIG.4

FIG.5

**FIG.6**

**FIG.7**

Europäisches
Patentamt

European
Patent Office

Office européen
des brevets

# EUROPEAN SEARCH REPORT

Application Number

EP 17 16 5507

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2007/201031 A1 (AXELROD NOEL [IL] ET AL) 30 August 2007 (2007-08-30) <br> * abstract; figures 1A, 1B, 4A * <br> * paragraphs [0013], [0034] - [0039] * <br> * the whole document * <br> ----- | 1-15 | INV. <br> A61B5/021 <br> A61B5/0215 <br> A61B5/00 |
| X <br> A | WO 97/24986 A2 (SUNLIGHT ULTRASOUND TECHNOLOGI [IL]; KANTOROVICH EDWARD [IL]) 17 July 1997 (1997-07-17) <br> * abstract * <br> * page 22, lines 5-23 * <br> ----- | 1 <br> 2-15 | |
| X <br> A | US 6 354 999 B1 (DGANY ELHANAN [IL] ET AL) 12 March 2002 (2002-03-12) <br> * column 4, lines 27-47 * <br> * the whole document * <br> ----- | 1 <br> 2-15 | |
| X | US 5 178 153 A (EINZIG ROBERT E [US]) 12 January 1993 (1993-01-12) <br> * abstract; claims 1, 2 * <br> * column 1, lines 50-60 * <br> * the whole document * <br> ----- | 1-15 | TECHNICAL FIELDS SEARCHED (IPC) <br><br> A61B |

The present search report has been drawn up for all claims

| Place of search | Date of completion of the search | Examiner |
|---|---|---|
| The Hague | 28 September 2017 | Furlan, Stéphane |

EPO FORM 1503 03.82 (P04C01)

## ANNEX TO THE EUROPEAN SEARCH REPORT
## ON EUROPEAN PATENT APPLICATION NO.

EP 17 16 5507

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2017

| Patent document cited in search report | | Publication date | Patent family member(s) | | Publication date |
|---|---|---|---|---|---|
| US 2007201031 | A1 | 30-08-2007 | US 2007201031 A1<br>WO 2007099532 A2 | | 30-08-2007<br>07-09-2007 |
| WO 9724986 | A2 | 17-07-1997 | AU 1169697 A<br>WO 9724986 A2 | | 01-08-1997<br>17-07-1997 |
| US 6354999 | B1 | 12-03-2002 | NONE | | |
| US 5178153 | A | 12-01-1993 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070201031 A1